# EUROPEAN PATENT APPLICATION

(11) **EP 2 241 634 A1**
(43) Date of publication of application: **20.10.2010**
(21) Application number: 09158093.6
(22) Date of filing: 16.04.2009
(51) Int. Cl.: C12Q 1/68

(54) **Diagnostic method and tools to predict the effiacy of targeted agents against IGF-1 pathway activation in cancer**

(71) Applicant: UNIVERSITE LIBRE DE BRUXELLES, 1050 Brussels (BE)
(72) Inventor: Sotiriou, Christos, 1080 Bruxelles (BE); Ignatiadis, Michail, 54644 Thessaloniki (GR)
(74) Representative: pronovem

(57) **Abstract**

The present invention relates to a gene set, kit and method to predict the efficacy of IGF-1 pathway inhibitors in cancer.

## Description

### Field of the invention

The present invention is related to methods and tools to assess prognosis and to predict the efficacy of targeted agents directed against the IGF-1 pathway in cancer.

### Background of the invention

Cancer, including breast cancer, lung cancer and colon cancer is a major threat to health in Western countries.

Breast cancer is the most common cancer in women in Western countries.

Breast cancer is an heterogenous disease that can be subdivided into subgroups depending on markers.

For instance, oestrogen receptor (ER) positive status is associated with a better outcome and may predict for a response to hormone treatments.

Her2 (ErB2; neu) is a member of the tyrosine-kinase family of Epithelial growth factor receptor (EGFR); its over-expression is associated with a poor progonstic.

However fortunately, Her-2-specific therapies, such as Trastuzumab (Herceptin) have been proved to be very active towards this subtype of breast cancer over-expressing Her2.

Lung cancer is responsible for more than 1 million deaths each year.

The main types of lung cancer are small cell lung carcinoma and non-small cell lung carcinoma. This distinction is important, because the treatment varies; non-small cell lung carcinoma (NSCLC) is sometimes treated with surgery, while small cell lung carcinoma (SCLC) usually responds better to chemotherapy and radiation.

In lung cancer, tyrosine-kinase inhibitors such as Gefitinib (Iressa) that targets EGFR, or other less-specific tyrosine-kinases inhibitors are used in second line of treatment.

There are several specific tyrosines-kinases inhibitors already available, or in clinical trials.

The Insulin-like Growth Factor 1 (IGF-1) Receptor is a transmembrane receptor that is activated by IGF-1 (and by the related growth factor IGF-2). It belongs to the large class of tyrosine kinase receptors.

The IGF-1 signalling pathway is of critical importance during normal development of mammary gland tissue inducing proliferation of epithelial cells and having a key role in inhibiting apoptosis.

This pathway and receptor are implicated in breast cancer by increasing the metastatic potential of the original tumour by inferring the ability to promote vascularisation.

Although in breast cancer, adjuvant tamoxifen reduces the risk of relapse in women with estrogen receptor (ER) positive disease by 41%, many women relapse in spite of adjuvant tamoxifen therapy.

Gene expression profiling studies have demonstrated that ER-positive disease is heterogeneous, with at least two distinguishable subtypes with markedly different outcomes under endocrine therapy. Further multiparameter expression studies have demonstrated that these distinct "luminal" subtypes are marked largely by differences in cellular proliferation.

There is a growing awareness that overactive signalling through growth factor receptor tyrosine kinases may contribute to endocrine resistance and the eventual development of recurrent disease following tamoxifen therapy.

Cross talk between the IGF-1R and Estrogen Receptor (ER) plays a critical role in tamoxifen resistance in breast cancer. Early studies demonstrated that increased plasma IGF levels identify women with an increased risk of relapse on adjuvant tamoxifen therapy. Preclinical experiments demonstrate that activation of IGF-1 pathway is associated with loss of progesterone receptor expression (PgR), a marker of the "high proliferative" and resistance to tamoxifen-induced apoptosis. More recent studies with animal models demonstrate that the establishment of a tamoxifen-resistant phenotype is marked by activation of the IGF-1R pathway.

Beside breast cancer, the IGF-1 pathway is implicated in several cancers. In some instances its anti-apoptotic properties allow cancerous cells to resist the cytotoxic properties of chemotherapeutic drugs or radiotherapy.

In some cancers, where EGFR inhibitors are being used to inhibit the EGFR signalling pathway, the activation of IGF-1 pathway confers resistance, allowing EGFR signalling to resume in the presence of a suitable inhibitor. This process is referred to as crosstalk between EGFR and IGF-1R.

In hope to improve outcome in early breast cancer antibodies specifically binding to IGF-1R were developed, selected for their capacity to target the IGF-1R pathway and are now tested in clinical trials.

For instance, the IGF-1R specific antibody CP-751,871 is currently in Phase III clinical trials for a form of lung cancer (NSCLC).

Besides specific antibodies, small molecules act are inhibitors of the IGF-1 pathway.

For instance IGF-1R antagonist BMS-7548077 binds to IGF-1R, preventing IGF-1 ligand binding and activation of IGF-1R-mediated signaling pathways.

INSM-18 is characterized as a "small molecule antagonist", and is a dual inhibitor of IGF-1R and Her2/neu in clinical trials (phase I/II in prostate cancer).

Several other molecules are either in preclinical development, or already in clinical trials.

There is therefore a need for diagnostics that can predict which patient will benefit from IGF-1 pathway-targeted therapies.

Conversely, the design of clinical trials, which include the selection and randomization of patients, may benefit for such diagnostic, as drug candidates developed to be specific for IGF-1 pathway should be given on subsets of patients having the same probability of response to the said treatment, preferably to patients having a predetermined increased probability of response to the drug candidate.

### Aims of the invention

The present invention aims to provide detection (diagnostic or prognosis) method and tools that do not present the drawbacks of the state of the art.

The present invention aims to provide methods and tools that determine a risk assessment of a tumor sample and/or improve prediction of this tumor sample response (and therefore patient response) to the administration of one or more anti IGF-1 signalling active compound(s) or drug(s) upon this tumor sample and consequently indentify the efficiency of this administration and the active compound(s) to this patient.

### Summary of the invention

The present invention relates to a gene set representing IGF-1 index.

Advantageously, the IGF-1 index is measured by mRNA quantification of selected genes.

The present invention relates to a gene or a gene set comprising (1), 2, 3, 4, 5, 6, 7, 8, 9, 10, 11, 12, 13, 14, 15, 16, 17, 18, 19, 20 or all the genes mentioned in Table 4 (representing IGF-1 signature).

The present invention also encompasses 1, 2, 3, 4, 5, 6, 7, 8, 9, 10, 11, 12, 13, 14, 15, 16, 17, 18, 19, 20 or all the genes of Table 5 (representing refined IGF-1 signature).

Preferably, the present invention also encompasses 1, 2, 3, 4, 5, 6, 7, 8, 9, 10, 11, 12, 13, 14, 15, 16, 17, 18, 19, 20 or all the genes of table 6 (representing the most refined IGF-1 signature).

Another aspect of the present invention is a diagnostic kit or device comprising capture probes being possibly fixed upon an (insoluble) solid support (such as a multiwall plate or a glass slide, preferably as an array), these capture probes detecting (by complementary base hybridization) specifically the expression of complementary target genes of the gene set of the invention, preferably 1, 2, 3, 4, 5, 6, 7, 8, 9, 10, 11, 12, 13, 14, 15, 16, 17, 18, 19, 20 or all the gene set of Tables 4, 5 or 6 (or the gene set representing the IGF-1 signature).

The present diagnostic kit comprising the gene set according to the invention further comprises other means for real time PCR analysis.

Preferably, the diagnostic kit or device of the invention comprises capture probes fixed upon a solid support and detecting specifically the expression of complementary target genes of the gene set of the invention and possibly others means for real time PCR.

Preferably, the invention provides for a kit or device being a computerized system comprising
- a bio-assay signature configured for detecting a gene expression from a tumor sample (a breast, colon or lung tumor sample, more preferably a breast tumor sample) based upon the gene set, the kit or devise according of the invention
- a processor module configured to calculate expression of these genes and to generate a risk assessment for the tumor (a breast tumor sample) sample.

Another aspect of the invention is a method for a prognosis (prognostic) of cancer in mammal subject, preferably in a human patient, which comprises the step of collecting a (breast) tumor sample, from the mammal subject and measuring gene expression in this (breast) tumor sample by putting into contact nucleotide sequences obtained from this (breast) tumor sample with the gene set or the kit or device according to any of the preceding claims and possibly generating a risk assessment for the (breast) tumor sample by designating the outcome of a IGF-1 specific regimen depending on the expression of the IGF-1 signature (using the gene sets of Tables 4-6).

Preferably, the method is for the prognosis (prognostic) of cancer selected from the group consisting of a breast, colon or lung cancer, affecting a human patient.

More preferably, the method is for the prognosis (prognostic) of (ER+ and Her2-) breast cancer.

The method is the measure of the expression profile of IGF-1 (signature), wherein a reduced expression IGF-1 signature represents an activation of the IGF-1 pathway and therefore an increased probability of benefit from IGF-1 targeted therapies.

By reduced expression, it is meant a low value of the IGF-1 signature, preferably lower than the median value in the patient population to be studied

Another aspect of the invention is related to a medical application of an active compound specifically inhibiting IGF-1 pathway for use in the treatment of (a breast, colon or lung cancer; more preferably a breast cancer; still more preferably a ER+ Her2- breast cancer) a cancer having an increased risk assessment as measured by the method of the invention and/or by the gene set of Tables 4 to 6 (as measured by a reduced expression of the IGF-1 signature, preferably wherein the values are below the median value of the patient population), or by the kit or device (derived thereof) of the invention.

By an active compound (or treatment or regimen) specifically inhibiting IGF-1 pathway, it is meant any treatment or active compound that may cause inhibition of IGF-1R signalling.

More precisely, the active compound specifically inhibiting IGF-1 pathway is based upon the administration of a sufficient amount of one or more monoclonal antibodies, preferably selected from the group consisting of CP-751, 871, IMC-A12, R1507, AMG-479, SCH-717454, AVE-1642, MK-0646, BIIB022 or a mixture thereof (being this active compound).

Alternatively, the active compound specifically inhibiting IGF-1 pathway is based upon the administration of a sufficient amount of one or more tyrosine kinase inhibitor(s), preferably selected from the group consisting of INSM-18, OSI-906, XL-228, NVP-ADW742, NVP-ADW541, AG-1024, BMS-536924, BMS-554417, BVP-51004 or mixture thereof (being this active compound).

The skilled person in the art may also identify or use other (specific or non-specific) treatment(s) or active compound(s) that target IGF-1 pathway, alone or in combination with conventional cancer active compounds, treatment protocols and treatment methods), such as chemotherapy, hormonotherapy or other targeted agents.

Another aspect of the invention is a medical application of chemotherapy or chemotherapeutic compound for use in the treatment of (preferably a breast, colon or lung cancer; more preferably a breast cancer; still more preferably a ER+ Her2- breast cancer) a cancer having an increased risk assessment as measured by a reduced expression of the IGF-1 signature (preferably wherein the values of the index of IGF-1 signature are below the median value of a patient population) as measured by the method of the invention and/or by the gene set of Tables 4-6 or by using kit or device derived thereof.

A related aspect of the present invention is the combination of IGF-1 pathway-specific treatment method (regimen) or active compound(s) with classical treatment(s), such as chemotherapy, hormonotherapy and/or targeted therapy (with administration of one or more other tyrosine-kinase inhibitor(s)) in identified patients with IGF-1 pathway activation (having reduced expression of the IGF-1 signature.

Another aspect of the invention is related to the monitoring, by the use of the gene set, (diagnostic) kit or device, of a patient disease evolution, receiving the above described IGF-1 related therapy or active compound(s) administration.

### Detailed description of the invention

### Patients and Methods

### Datasets

Publicly available clinical and genomic data from the NKI (van de Vijver, et al. (N Engl J Med 2002; 347(25):1999-2009.), Veridex (Wang Y, et al. (Lancet 2005; 365 (9460) : 671-679*.*), TRANSBIG (Desmedt C, et al. (Clin Cancer Res 2007; 13(11):3207-3214*),* MAINZ (Schmidt M, et al. (Cancer Res. 2008; 68(13):5405-5413*.)* and a combined Tamoxifen-only treated dataset (Loi S, et al. (J Clin Oncol 2007; 25(10):1239-1246*.* and Haibe-Kains B, et al. (BMC Genomics 2008; 9:394*)* were used for the present analysis.

### Molecular subtypes

Patients were categorized as ER-/HER2-, HER2+ and ER+/HER2- based on the breast cancer molecular modules as previously defined by Desmedt C, et al. (Clin Cancer Res 2008; 14(16):5158-5165).

The present analysis was confined to the ER+/HER2- patients. These were further divided into high (luminal B) and low (luminal A) proliferative subtypes using the Genomic Grade Index (GGI).

The association between IGF-1 signature and clinical outcome was performed using several publicly available datasets including 590 untreated and 349 tamoxifen-only treated ER+/HER2- early BC patients.

### IGF-1 signature

The inventors have developed this IGF-1 signature based on the prototype-based coexpression modules approach (Desmedt et al.). To identify the IGF-1 signature the inventors selected genes whose expression was significantly correlated to the prototype IGF-1, more than to other prototypes namely ER, HER2 and AURKA using a similar approach (Desmedt et al.). IGF-1 signature score for a specific data set was computed for each patient as signature score =wᵢxᵢ=fᵢⱼwᵢⱼ
where xᵢ is the expression of a gene in the signature that is present in the data set platform, and wᵢ is either +1 or -1 depending on the sign of the association with the prototype IGF-1.

IGF-1 signature (activated vs inhibited): Patients were categorized as having an activated vs inhibited IGF-1 signature if they had values equal or less vs higher than the IGF-1 signature median value in the ER+/HER2- subgroup for each one of the dataset, separately. Ki67mRNA (high vs low): Patients were categorized as having a high or low Ki67mRNA expression, if they had values higher vs equal or less than the median value of the Ki67mRNA in the ER+/HER2- subgroup for each one of the dataset, separately.
PRmRNA (high vs low): Patients were categorized as having a high or low PRmRNA expression, if they had values higher vs equal or less than the median value of the PRmRNA in the ER+/HER2- subgroup for each one of the dataset, separately. 70-gene signature (poor vs good prognosis): Patients were categorized as having a poor vs good prognosis signature if they had values higher vs equal or less than the 70-gene signature median value in the ER+/HER2- subgroup for each one of the dataset, separately.

GGI (high *vs* low) A high *vs* low GGI value corresponded to values higher vs equal or less than the midpoint between the average gene expression grade index values from histology grade 1 and histology grade 3 tumors in the ER+/HER2- subgroup for each one of the dataset, separately.

### Statistical Analysis

Summary descriptive statistics were expressed as mean (standard deviation [SD]) or percent, as appropriate. Continuous variables were compared between different groups with Kruskal-Wallis test as appropriate, and categoric data were compared between different groups with Fisher's exact test. The time from study entry until the day of the first evidence of distant disease recurrence (distant metastasis-free survival [DMFS]) was the main dependent variables of the study. DMFS curves for subgroups of patients were constructed using the Kaplan-Meier product limit estimate method and were compared by the log-rank test in order to provide a univariate assessment of the prognostic value of selected clinical and genomic risk factors.

Clinical, pathological and genomic factors known to be associated with prognosis like age (≤50 *vs* >50), tumor size (T2-3 *vs* T1), nodal infiltration (yes *vs* no), histology grade (3 *vs* 2 and 1), (3 and 2 *vs* 1), Ki67mRNA (high vs low), PRmRNA (high vs low), GGI (high *vs* low), 70-gene signature (high vs low), IGF-1 signature (activated vs inhibited), Tamoxifen (yes vs no, corresponding to tamoxifen-only treated vs untreated patients) were tested in univariate analysis.

Variables that were found to be significant at the univariate screen were then entered in a stepwise multivariate Cox proportional hazards regression model in order to identify those with independent prognostic information. The 70-gene signature and the GGI have similar prognostic performance.

### The inventors tested two multivariate models:

Multivariate model 1 on systemically untreated and tamoxifen only treated patients with ER+/HER2- early BC including tumor size, nodal infiltration, histology grade, Ki67mRNA, PRmRNA, GGI and IGF-1 signature
Multivariate model 2 on systemically untreated and tamoxifen only treated patients with ER+/HER2- early BC including tumor size, nodal infiltration, histology grade, Ki67mRNA, PRmRNA, 70-gene signature and IGF-1 signature Entry into and removal from the model were set at 5% and 10%, respectively.

All statistical tests were performed at the 5% level of significance. SPSS, version 15 (SPSS Inc, Chicago, IL), statistical software was used for the analysis.

### Results

### Patients Characteristics

Table 1 describes the characteristics of the patients with ER+/HER2- that was used to evaluate the IGF-1 signature of the invention. A higher proportion of patients treated with tamoxifen-only as compared to untreated patients were older than 50 years, had tumors > 2cm and infiltrated axillary lymph nodes. Patients with an activated IGF-1 signature were significantly associated with tumor size larger than 2 cm, histology grade 3 tumors, high Ki67mRNA, high GGI and high 70-gene signature.

**Table 1: ER+/HER2- Patient Characteristics and IGF-1 pathway activation**

| **Total** | **IGF1 activated** **N (%)** **468 (100)** | **IGF1 inhibited** **N (%)** **471 (100)** | ***P*** |
|---|---|---|---|
| **Age** | | | |
| **≤ 50** | **150 (32)** | **166 (35)** | **0.230** |
| **> 50** | **237 (51 )** | **220 (47)** | |
| **Unknown** | **81 (17)** | **85 (18)** | |
| **Tumor Size** | | | |
| **≤ 2** | **250 (53)** | **312 (66)** | **<0.001** |
| **> 2** | **216 (46)** | **153 (33)** | |
| **Unknown** | **2(1)** | **6(1)** | |
| **Nodal status** | | | |
| **Negative** | **354 (75)** | **366 (78)** | **0.288** |
| **Positive** | **112 (24)** | **98(21)** | |
| **Unknown** | **2(1)** | **7(1)** | |
| **Histology Grade** | | | |
| **1** | **60 (13)** | **115 (24)** | **<0.001** |
| **2** | **203 (43)** | **210 (45)** | |
| **3** | **157 (34)** | **76 (16)** | |
| **Unknown** | **48 (10)** | **70 (15)** | |
| **Ki67mRNA** | | | |
| **Low** | 1**76 (38)** | **294 (62)** | **<0.001** |
| **High** | **292 (62)** | **177 (38)** | |
| **PRmRNA** | | | |
| **Low** | **241 (52)** | **224 (48)** | **0.228** |
| **High** | **227 (48)** | **247 (52)** | |
| **GGI** | | | |
| **Low** | **172 (37)** | **347 (74)** | **<0.001** |
| **High** | **296 (63)** | **124(26)** | |
| **70-gene signature** | | | |
| **Low** | **176 (38)** | **298 (63)** | **<0.001** |
| **High** | **292 (62)** | **173 (37)** | |

### Univariate and Multivariate Analysis

In univariate analysis, tumor size larger than 2 cm, histology grade 2 and 3, low expression of PRmRNA, high expression of Ki67mRNA, high risk gene expression profile based on GGI and on the 70-gene signature and an activated IGF-1 signature were associated with significantly shorter DMFS in the untreated and tamoxifen only treated patients with ER+/HER2- early BC (Table2).

The inventors observed that IGF-1 signature expression is negatively correlated with the expression of proliferation genes.

The inventors observed that IGF-1 signature expression is negatively correlated with another IGF-1 signature developed in vitro by stimulating MCF-7 cells with IGF-1.

The inventors observed that IGF-1 signature expression is correlated with worse survival outcome of patient

Therefore, the inventors conclude that low expression values of the IGF-1 signature they developed is associated with an activated IGF-1 pathway in patients, preferably in human patients suffering from a cancer still more preferably selected from the group consisting of breast, colon and lung cancer)

**Table 2: Univariate analysis on systemically untreated and tamoxifen only treated women with ER+/HER2- early BC**

| **Distant-Metastasis Free Survival** | | **HR (95% CI)** | ***P*** |
|---|---|---|---|
| **Age (≤50 vs >50)** | | **0.967 (0.739-1.266)** | **0.809** |
| **Tumor Size (T2-3 vs T1)** | | **1.433 (1.115-1.842)** | **0.005** |
| **Axillary Nodal status (pos vs neg)** | | **1.205 (0.900-1.612)** | **0.211** |
| **Histology Grade** | **(3 and 2 vs 1)** | **2.713 (1.728-4.261)** | **<0.001** |
| | **(3 vs 2 and 1)** | **2.120 (1.613-2.787)** | **<0.001** |
| **Ki67mRNA (High vs low)** | | **1.970 (1.520-2.554)** | **<0.001** |
| **PRmRNA (High vs low)** | | **0.479 (0.369-0.622)** | **<0.001** |
| **GGl (High vs Low)** | | **2.891 (2.223-3.761)** | **<0.001** |
| **70 Gene Signature (High vs Low)** | | **2.520 (1.927-3.294)** | **<0.001** |
| **IGF1 Module (activated vs inhibited)** | | **2.039 (1.573-2.644)** | **<0.001** |
| **Tamoxifen (yes vs no )** | | **0.904 (0.789-1.037)** | **0.149** |
| **IGF1 Signature*Tamoxifen** | | **1.203 (1.031-1.403)** | **0.019** |

In multivariate analysis (multivariate model 1) tumor size larger than 2 cm, histology grade 2 and 3, low expression of PRmRNA, high GGI and an activated IGF-1 signature were independently associated with decreased DMFS (Table 3).

**Table 3: Multivariate analysis**

| **Distant Metastasis free Survival** | **HR (95% Cl)** | ***P*** |
|---|---|---|
| **Tumor Size (T2-3 vs T1)** | **1.351 (1.029-1.775)** | **0.030** |
| **Histology Grade (3 and 2 vs 1)** | **1.718 (1.071-2.755)** | **0.025** |
| **PRmRNA (High vs low)** | **0.573 (0.430-0.763)** | **<0.001** |
| **GGl (High vs Low)** | **1.908 (1.382-2.634)** | **<0.001** |
| **IGF1 Module (activated vs inhibited)** | **1.483 (1.086-2.024)** | **0.013** |

In multivariate model 2 that included the 70-gene signature instead the GGI, low expression of PRmRNA, high risk gene expression profile based on the 70-gene signature and an activated IGF-1 signature were independent prognostic factors for decreased DMFS.

The complete IGF-1 signature is composed of 327 genes (Table 4).

| Entrez.ID | Gene symbol | coef | Entrez.ID | Gene symbol | coef | Entrez.ID | Gene symbol | coef | Entrez.ID | Gene symbol | coef | Entrez.ID | Gene symbol | coef |
|---|---|---|---|---|---|---|---|---|---|---|---|---|---|---|
| 3479 | IGF1 | 1 | 2149 | F2R | 1 | 113791 | NA | 1 | 4313 | MMP2 | 1 | 84823 | LMNB2 | -1 |
| 10186 | LHFP | 1 | 55143 | CDCA8 | -1 | 8438 | RAD54L | -1 | 1036 | CDO1 | 1 | 716 | C1S | 1 |
| 7373 | COL14A1 | 1 | 2273 | FHL1 | 1 | 6196 | RPS6KA2 | 1 | 9445 | ITM2B | 1 | 1281 | COL3A1 | 1 |
| 8404 | SPARCL1 | 1 | 9839 | ZFHX1B | 1 | 3487 | IGFBP4 | 1 | 9133 | CCNB2 | -1 | 10897 | YIF1A | -1 |
| 219654 | C10orf56 | 1 | 991 | CDC20 | -1 | 1293 | COL6A3 | 1 | 150 | ADRA2A | 1 | 6366 | CCL21 | 1 |
| 23768 | FLRT2 | 1 | 948 | CD36 | 1 | 23037 | PDZD2 | 1 | 11004 | KIF2C | -1 | 2617 | GARS | -1 |
| 730 | C7 | 1 | 23397 | NCAPH | -1 | 6578 | SLCO2A1 | 1 | 7167 | TP11 | -1 | 11339 | OIP5 | -1 |
| 125 | ADH1B | 1 | 1058 | CENPA | -1 | 57161 | PELI2 | 1 | 3590 | IL11RA | 1 | 54810 | GIPC2 | 1 |
| 80310 | PDGFD | 1 | 11167 | FSTL1 | 1 | 60468 | BACH2 | 1 | 5950 | RBP4 | 1 | 5204 | PFDN5 | 1 |
| 8483 | CILP | 1 | 891 | CCNB1 | -1 | 22948 | CCT5 | -1 | 2305 | FOXM1 | -1 | 54832 | VPS13C | 1 |
| 857 | CAV1 | 1 | 2487 | FRZB | 1 | 890 | CCNA2 | -1 | 79652 | C16orf30 | 1 | 55629 | PNRC2 | 1 |
| 4222 | MEOX1 | 1 | 687 | KLF9 | 1 | 51155 | HN1 | -1 | | PKMYT1 | -1 | 10460 | TACC3 | -1 |
| 4969 | OGN | 1 | 347 | APOD | 1 | 2788 | GNG7 | 1 | 9088 7043 | TGFB3 | 1 | 993 | CDC25A | -1 |
| 2205 | FCER1A | 1 | 10462 | CLEC10A | 1 | 4171 | MCM2 | -1 | 83468 | GLT8D2 | 1 | 10544 | PROCR | 1 |
| 5627 | PROS1 | 1 | 10742 | RAI2 | 1 | 27123 | DKK2 | 1 | 26112 | CCDC69 | 1 | 80308 | FLAD1 | -1 |
| 8639 | AOC3 | 1 | 55355 | NA | -1 | 65997 | RASL11B | 1 | 4212 | MEIS2 | 1 | 2252 | FGF7 | 1 |
| 7060 | THBS4 | 1 | 26468 | LHX6 | 1 | 10059 | DNM1L | -1 | 5587 | PRKD1 | 1 | 55084 | NA | 1 |
| 6424 | SFRP4 | 1 | 7422 | VEGFA | -1 | 4282 | MIF | -1 | 10365 | KLF2 | 1 | | LDHA | -1 |
| 9936 | CD302 | 1 | 9055 | PRC1 | -1 | 54988 | NA | 1 | 27244 | SESN1 | 1 | 3939 1959 | EGR2 | 1 |
| 57088 | PLSCR4 | 1 | 24137 | KIF4A | -1 | 55970 | GNG12 | 1 | 6241 | RRM2 | -1 | 55159 | RFWD3 | -1 |
| 10252 | SPRY1 | 1 | 332 | BIRC5 | -1 | 1503 | CTPS | -1 | 54478 | FAM64A | -1 | 4094 | MAF | 1 |
| 83700 | JAM3 | 1 | 7049 | TGFBR3 | 1 | 667 | DST | 1 | 4839 | NOL1 | -1 | 54491 | FAM105A | 1 |
| 9353 | SLIT2 | 1 | 9547 | CXCL 14 | 1 | 3512 | IGJ | 1 | 55830 | GLT8D1 | 1 | 7272 | TTK | -1 |
| 9590 | AKAP12 | 1 | 2152 | F3 | 1 | 10212 | DDX39 | -1 | 7123 | CLEC3B | 1 | 22827 | NA | -1 |
| 9023 | CH25H | 1 | 79776 | ZFHX4 | 1 | 51659 | GINS2 | -1 | 9146 | HGS | -1 | 5649 | RELN | 1 |
| 10161 | P2RY5 | 1 | 51474 | LIMAL | 1 | 10512 | SEMA3C | 1 | 1843 | DUSP1 | 1 | 51646 | YPEL5 | 1 |
| 8434 | RECK | 1 | 1805 | DPT | 1 | 5532 | PPP3CB | 1 | 4060 | LUM | 1 | 2353 | FOS | 1 |
| 23460 | ABCA6 | 1 | 9700 | ESPL1 | -1 | 5531 | PPP4C | -1 | 10535 | RNASEH2A | -1 | 4915 | NTRK2 | 1 |
| 5243 | ABCB1 | 1 | 9319 | TRIP13 | -1 | 1389 | CREBL2 | 1 | 9787 | DLG7 | -1 | 89795 | NAV3 | 1 |
| 4675 | NAP1L3 | 1 | 51170 | HSD17B11 | 1 | 23398 | PPWD1 | 1 | 6182 | MRPL12 | -1 | 54821 | NA | -1 |
| 1910 | EDNRB | 1 | 9232 | PTTG1 | -1 | 10615 | SPAG5 | -1 | 11275 | KLHL2 | 1 | 55857 | C20orf19 | 1 |
| 22974 | TPX2 | -1 | 115207 | KCTD12 | 1 | 23594 | ORC6L | -1 | 29127 | RACGAP1 | -1 | 6867 | TACC1 | 1 |
| 1003 | CDH5 | 1 | 56898 | BDH2 | 1 | 301 | ANXA1 | 1 | 26137 | ZBTB20 | 1 | 3014 | H2AFX | -1 |
| 6414 | SEPP1 | 1 | 8914 | TIMELESS | -1 | 1776 | DNASE1L3 | 1 | 10574 | CCT7 | -1 | 26872 | STEAP1 | 1 |
| 3426 | CFI | 1 | 9358 | ITGBL1 | 1 | 5547 | PRCP | 1 | 1164 | CKS2 | -1 | 2191 | FAP | 1 |
| 1675 | CFD | 1 | 55901 | THSD1 | 1 | 79846 | NA | 1 | 16 | AARS | -1 | 8833 | GMPS | -1 |
| 23090 | ZNF423 | 1 | 11188 | NISCH | 1 | 4881 | NPR1 | 1 | 54816 | SUHW4 | 1 | 3161 | HMMR | -1 |
| 1511 | CTSG | 1 | 51306 | C5orf5 | 1 | 66036 | MTMR9 | 1 | 23371 | TENC1 | 1 | 9037 | SEMA5A | 1 |
| 56920 | SEMA3G | 1 | 4147 | MATN2 | 1 | 23047 | APRIN | 1 | 26040 | SETBP1 | 1 | 10134 | BCAP31 | -1 |
| 1842 | ECM2 | 1 | 7704 | ZBTB16 | 1 | 4085 | MAD2L1 | -1 | 22915 | MMRN1 | 1 | 79971 | GPR177 | 1 |
| 5346 | PLIN | 1 | 7083 | TK1 | -1 | 7298 | TYMS | -1 | 641 | BLM | -1 | 54360 | CYTL1 | 1 |
| 2669 | GEM | 1 | 54861 | SNRK | 1 | 11130 | ZWINT | -1 | 201134 | CCDC46 | 1 | 55717 | BRWD2 | 1 |
| 1102 | RCBTB2 | 1 | 23179 | RGL1 | 1 | 4173 | MCM4 | -1 | 3068 | HDGF | -1 | 5708 | PSMD2 | -1 |
| 6925 | TCF4 | 1 | 2331 | FMOD | 1 | 9627 | SNCAIP | 1 | 55872 | PBK | -1 | 6863 | TAC1 | 1 |
| 3679 | ITGA7 | 1 | 1453 | CSNK1D | -1 | 26289 | AK5 | 1 | 4674 | NAP1 L2 | 1 | 3838 | KPNA2 | -1 |
| 9737 | GPRASP1 | 1 | 4330 | MN1 | 1 | 55526 | DHTKD1 | -1 | 55839 | CENPN | -1 | 4211 | MEIS1 | 1 |
| 5654 | HTRA1 | 1 | 10112 | KIF20A | -1 | 83989 | C5orf21 | 1 | 1808 | DPYSL2 | 1 | 1033 | CDKN3 | -1 |
| 10580 | SORBS1 | 1 | 2615 | LRRC32 | 1 | 1149 | CIDEA | 1 | 10328 | COX4NB | -1 | 10631 | POSTN | 1 |
| 11065 | UBE2C | -1 | 79019 | CENPM | -1 | 10491 | CRTAP | 1 | 3820 | KLRB1 | 1 | 58486 | ZBED5 | 1 |
| 5468 | PPARG | 1 | 55165 | CEP55 | -1 | 2122 | EVI1 | 1 | 54892 | NCAPG2 | -1 | 51232 | CRIM1 | 1 |
| 10234 | LRRC17 | 1 | 23479 | ISCU | 1 | 51669 | TMEM66 | 1 | 1811 | SLC26A3 | 1 | 6653 | SORL1 | 1 |
| 10752 | CHL1 | 1 | 4605 | MYBL2 | -1 | 56944 | OLFML3 | 1 | 5159 | PDGFRB | 1 | 5176 | SERPINF1 | 1 |
| 10788 | IQGAP2 | 1 | 10231 | DSCR1L1 | 1 | 50650 | ARHGEF3 | 1 | 1163 | CKS1B | -1 | 9793 | CKAP5 | -1 |
| 3489 | IGFBP6 | 1 | 1958 | EGR1 | 1 | 6363 | CCL19 | 1 | 2690 | GHR | 1 | 54512 | EXOSC4 | -1 |
| 8322 | FZD4 | 1 | 51512 | GTSE1 | -1 | 64321 | SOX17 | 1 | 51514 | DTL | -1 | 6513 | SLC2A1 | -1 |
| 1359 | CPA3 | 1 | 395 | ARHGAP6 | 1 | 9833 | MELK | -1 | 8260 | ARD1A | -1 | 23214 | XPO6 | -1 |
| 2277 | FIGF | 1 | 4751 | NEK2 | -1 | 1072 | CFL1 | -1 | 56034 | PDGFC | 1 | 389136 | VGLL3 | 1 |
| 862 | RUNX1T1 | 1 | 8532 | CPZ | 1 | 215 | ABCD1 | -1 | 2 | A2M | 1 | 51601 | LIPT1 | 1 |
| 63923 | TNN | 1 | 9156 | EXO1 | -1 | 3991 | LIPE | 1 | 79365 | BHLHB3 | 1 | 54845 | RBM35A | -1 |
| 1601 | DAB2 | 1 | 7153 | TOP2A | -1 | 2199 | FBLN2 | 1 | 54819 | ZCCHC10 | 1 | 2123 | EVI2A | 1 |
| 4958 | OMD | 1 | 23024 | PDZRN3 | 1 | 7371 | UCK2 | -1 | 3028 | HSD17B10 | -1 | 912 | CD1D | 1 |
| 4081 | MAB21L1 | 1 | 2206 | MS4A2 | 1 | 54997 | TESC | 1 | 11081 | KERA | 1 | 22841 | RAB11FIP2 | 1 |
| 5157 | PDGFRL | 1 | 126 | ADH1C | 1 | 28984 | NA | 1 | 8839 | WISP2 | 1 | 990 | CDC6 | -1 |
| 5156 | PDGFRA | 1 | 10144 | FAM13A1 | 1 | 7538 | ZFP36 | 1 | | | | 23077 | MYCBP2 | 1 |

The inventors further eliminated from the signature genes related to other known pathways (such as estrogen receptor, HER2 signalling, proliferation,) and developed a smaller signature of 142 genes that more specifically describes IGF-1 pathway.

This refined (smaller) signature comprises the IGF-1 gene, 133 genes that were positively correlated with IGF-1 and 8 genes that were negatively correlated with IGF-1. (Table 5)

**Table 5: Refined IGF-1 signature**

| Entrez.ID | Gene symbol | coef | Entrez.ID | Gene symbol | coef | Entrez.ID | Gene symbol | coef | Entrez.ID | Gene symbol | coef | Entrez.ID | Gene symbol | coef |
|---|---|---|---|---|---|---|---|---|---|---|---|---|---|---|
| 3479 | IGF1 | 1 | 1675 | CFD | | 4212 | MEIS2 | 1 | 1843 | DUSP1 | 1 | 716 | C1S | 1 |
| 10186 | LHFP | 1 | 1511 | CTSG | 1 | 9839 | ZFHX1B | 1 | 4060 | LUM | 1 | 10897 | YIF1A | -1 |
| 7373 | COL14A1 | 1 | 1842 | ECM2 | 1 | 948 | CD36 | 1 | 11275 | KLHL2 | 1 | 6366 | CCL21 | 1 |
| 8404 | SPARCL1 | 1 | 5346 | PLIN | 1 | 11167 | FSTL1 | 1 | 6196 | RPS6KA2 | 1 | 54810 | GIPC2 | 1 |
| 219654 | C10orf56 | 1 | 2669 | GEM | 1 | 347 | APOD | 1 | 1293 | COL6A3 | 1 | 10544 | PROCR | 1 |
| 23768 | FLRT2 | 1 | 1102 | RCBTB2 | 1 | 10462 | CLEC10A | 1 | 23037 | PDZD2 | 1 | 2252 | FGF7 | 1 |
| 730 | C7 | 1 | 6925 | TCF4 | 1 | 26468 | LHX6 | 1 | 6578 | SLCO2A1 | 1 | 55084 | NA | 1 |
| 125 | ADH1B | 1 | 3679 | ITGA7 | 1 | 7049 | TGFBR3 | 1 | 4282 | MIF | -1 | 1959 | EGR2 | 1 |
| 8483 | CILP | 1 | 9737 | GPRASP1 | 1 | 9547 | CXCL14 | 1 | 667 | DST | 1 | 4094 | MAF | 1 |
| 857 | CAV1 | 1 | 5654 | HTRA1 | 1 | 2152 | F3 | 1 | 3512 | IGJ | 1 | 5649 | RELN | 1 |
| 4222 | MEOX1 | 1 | 10580 | SORBS1 | 1 | 1805 | DPT | 1 | 5531 | PPP4C | -1 | 6867 | TACC1 | 1 |
| 4969 | OGN | 1 | 5468 | PPARG | 1 | 51170 | HSD17B11 | 1 | 23398 | PPWD1 | 1 | 10134 | BCAP31 | -1 |
| 2205 | FCER1A | 1 | 10752 | CHL1 | 1 | 115207 | KCTD12 | 1 | 1776 | DNASE1L3 | 1 | 54360 | CYTL1 | 1 |
| 5627 | PROS1 | 1 | 10788 | IQGAP2 | 1 | 4147 | MATN2 | 1 | 5547 | PRCP | 1 | 4211 | MEIS1 | 1 |
| 8639 | AOC3 | 1 | 3489 | IGFBP6 | 1 | 54861 | SNRK | 1 | 4881 | NPR1 | 1 | 58486 | ZBED5 | 1 |
| 7060 | THB54 | 1 | 8322 | FZD4 | 1 | 23179 | RGL1 | 1 | 9627 | SNCAIP | 1 | 389136 | VGLL3 | 1 |
| 9936 | CD302 | 1 | 1359 | CPA3 | 1 | 1453 | CSNK1D | -1 | 1149 | CIDEA | 1 | 51601 | LIPT1 | 1 |
| 10252 | SPRY1 | 1 | 2277 | FIGF | 1 | 2615 | LRRC32 | 1 | 2122 | EVIL | 1 | 2123 | EVI2A | 1 |
| 83700 | JAMB | 1 | 862 | RUNX1T1 | 1 | 10231 | DSCR1L1 | 1 | 6363 | CCL19 | 1 | 912 | CD1D | 1 |
| 9353 | SLIT2 | 1 | 63923 | TNN | 1 | 1958 | EGR1 | 1 | 64321 | SOX17 | 1 | 23077 | MYCBP2 | 1 |
| 9590 | AKAP12 | 1 | 1601 | DAB2 | 1 | 395 | ARHGAP6 | 1 | 215 | ABCD1 | -1 | 8644 | AKR1C3 | 1 |
| 9023 | CH25H | 1 | 4081 | MAB21L1 | 1 | 23024 | PDZRN3 | 1 | 2199 | FBLN2 | 1 | 3820 | KLRB1 | 1 |
| 10161 | P2RY5 | 1 | 5157 | PDGFRL | 1 | 126 | ADH1C | 1 | 28984 | NA | 1 | 5159 | PDGFRB | 1 |
| 8434 | RECK | 1 | 5156 | PDGFRA | 1 | 10144 | FAM13A1 | 1 | 7538 | ZFP36 | 1 | 2 | A2M | 1 |
| 23460 | ABCA6 | 1 | 4313 | MMP2 | 1 | 4311 | MME | 1 | 10574 | CCT7 | -1 | 79365 | BHLHB3 | 1 |
| 5243 | ABCB1 | 1 | 1036 | CDO1 | 1 | 5587 | PRKD1 | 1 | 22915 | MMRN1 | 1 | 3426 | CFI | 1 |
| 1910 | EDNRB | 1 | 3590 | IL11RA | 1 | 10365 | KLF2 | 1 | 201134 | CCDC46 | 1 | 1808 | DPYSL2 | 1 |
| 1003 | CDH5 | 1 | 5950 | RBP4 | 1 | 7123 | CLEC3B | 1 | 4674 | NAP1L2 | 1 | 9146 | HGS | -1 |
| 6414 | SEPP1 | 1 | 26112 | CCDC69 | 1 | | | | | | | | | |

The inventors further used the data from Creighton et al. and performed the analysis of gene set enrichment and obtained a third signature that encompasses the genes more closely resembling to IGF-1 (Table 6).

**Table 6; most-refined signature**

| Entrez ID | gene symbol | coef | Entrez ID | gene symbol | coef | Entrez ID | gene symbol | coef | Entrez ID | gene symbol | coef |
|---|---|---|---|---|---|---|---|---|---|---|---|
| 6414 | SEPP1 | 1 | 10144 | FAM13A1 | 1 | 6867 | TACC1 | 1 | 1601 | DAB2 | 1 |
| 4311 | MME | 1 | 9353 | SLIT2 | 1 | 23024 | PDZRN3 | 1 | 5587 | PRKD1 | 1 |
| 51170 | HSD17B11 | 1 | 9936 | CD302 | 1 | 9627 | SNCAIP | 1 | 23398 | PPWD1 | 1 |
| 857 | CAV1 | 1 | 8644 | AKR1C3 | 1 | 23037 | PDZD2 | 1 | 26468 | LHX6 | 1 |
| 4147 | MATN2 | 1 | 5547 | PRCP | 1 | 2122 | EVI1 | 1 | 10252 | SPRY1 | 1 |
| 1102 | RCBTB2 | 1 | 51601 | LIPT1 | 1 | 5627 | PROS1 | 1 | 1293 | COL6A3 | 1 |

The inventors then tested the prognostic potential of the 142-gene signature of Table 5 on untreated patients with Er+/Her2- early breast cancer.

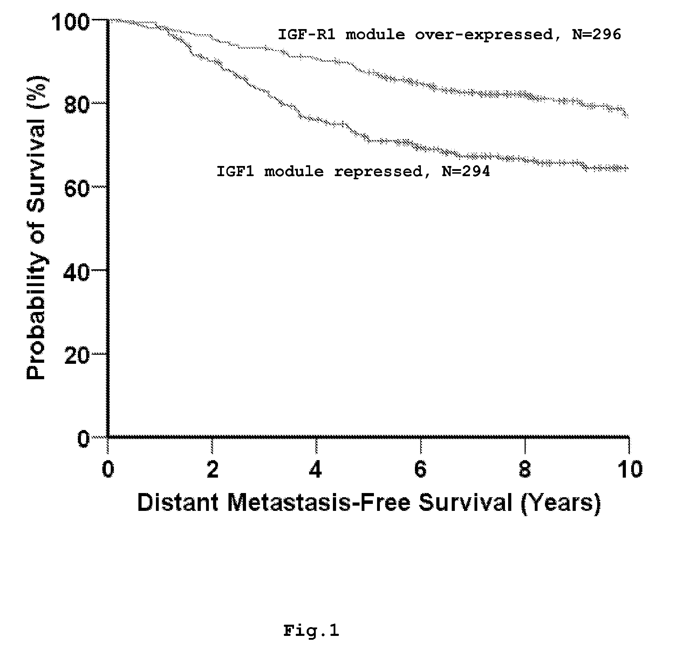

As shown in Figure 1, when the IGF-1 signature is over-expressed, the distant metastasis-free survival is clearly better (p<0.001). About half of the patients have over-expressed IGF-1 signature.

Clearly, reduced expression of the IGF-1 signature is associated with poor prognosis in untreated patients with Er+/Her2- early breast cancer.

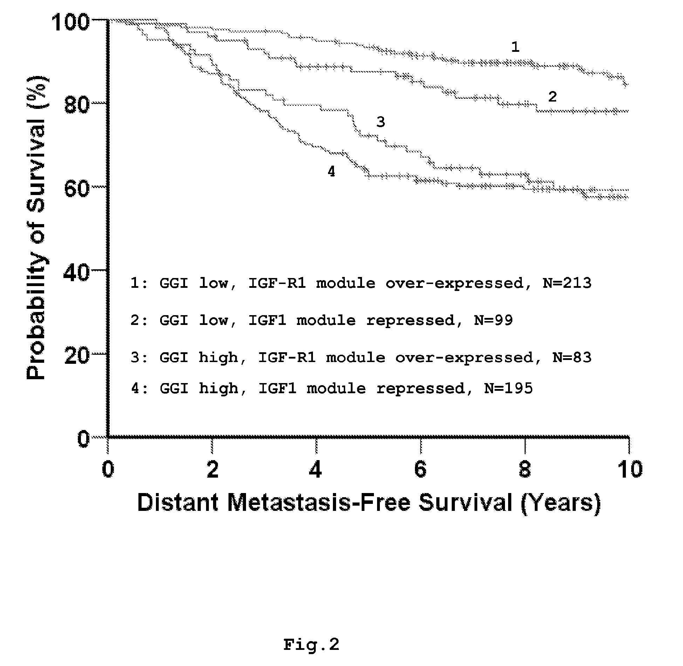

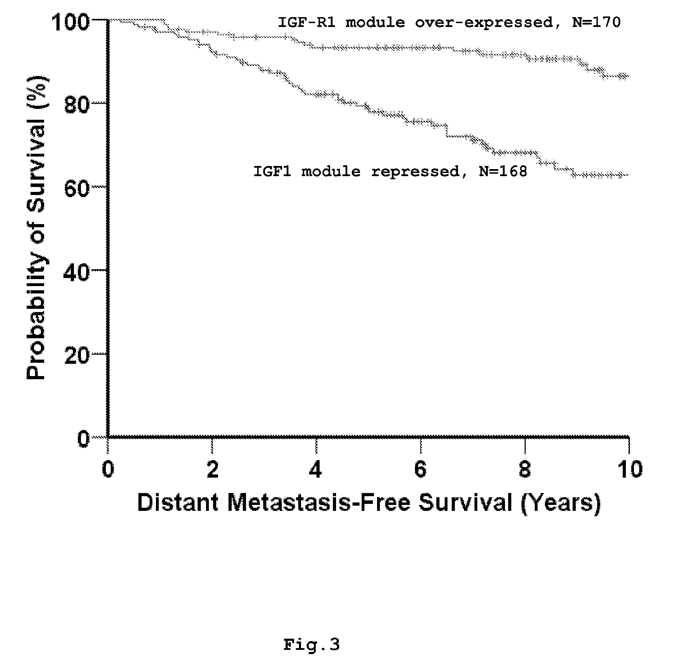

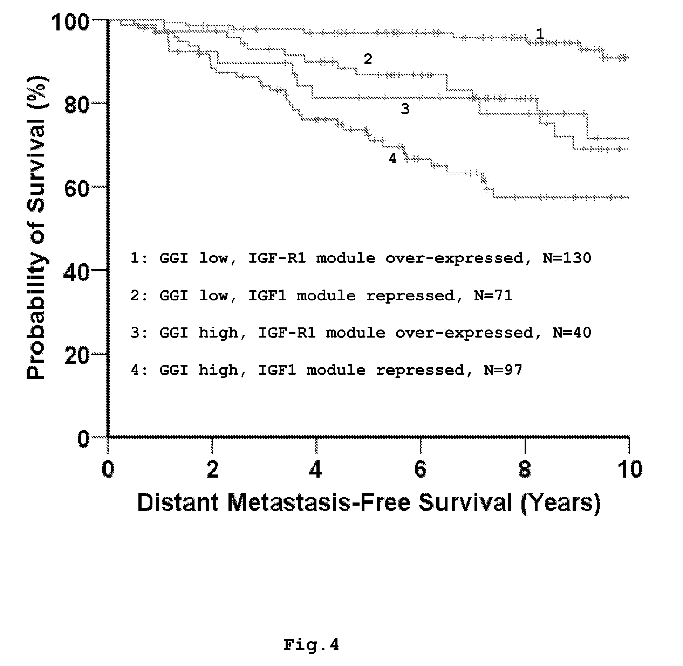

The inventors further characterize distant metastasis-free survival in function of both the GGI value (proliferation index) and the IGF-1 signature (Figure 2) in Er+/Her2- breast cancer untreated patients.

As previously published, high genomic grade predicted a poor outcome.

The reduced expression of the IGF-1 signature predicted a poor outcome.

The inventors performed the same studies in tamoxifen-treated patients.

The reduced expression of the IGF-1 signature predicted a poor outcome also in Tamoxifen-treated patients (ER+ and Her2-).

When combining both the GGI and IGF-1 signatures in Tamoxifen-treated patients, the inventors observed a better prognosis of patients over-expressing the IGF-1 signature (inactive IGF-1 pathway), especially in low-GGI patients (p<0.001).

Advantageously, in multivariate model this IGF-1 signature is independent of GGI or other proliferation index.

The inventors further conclude that IGF-1 signature is a valuable diagnostic tool to predict the outcome of patients whether untreated or treated with Tamoxifen.

More generally, the inventors further conclude that the reduced expression of IGF-1 signature represents an increased risk of relapse as it is related to activated IGF-1 pathway.

Therefore, targeted agents directed against the IGF-1 signaling pathway can be administrated to patients having a reduced expression of the IGF-1 signature (activated IGF-1 pathway), whether in a monotherapy or in combination with radiotherapy, chemotherapy, hormonotherapy or other targeted agents

Finally, the inventors used this IGF-1 signature to monitor the effect(s) of a treatment applied to a cancer patient.

This last application of monitoring the effect of a treatment upon a human patient; is especially valuable for patients treated with these IGF-1 specific drugs (active compounds).

## Claims

1. A gene or gene set comprising 1, 2, 3, 4, 5, 6, 7, 8, 9, 10, 11, 12, 13, 14, 15, 16, 17, 18, 19, 20 or all the genes mentioned in Table 6.

2. The gene set of claim 1 further comprising 1, 2, 3, 4, 5, 6, 7, 8, 9, 10, 11, 12, 13, 14, 15, 16, 17, 18, 19, 20 or all the genes of Table 5 (not present in Table 6).

3. The gene set of claim 2 further comprising 1, 2, 3, 4, 5, 6, 7, 8, 9, 10, 11, 12, 13, 14, 15, 16, 17, 18, 19, 20 or all the genes of table 4 (not present in Table 5).

4. A diagnostic kit or device comprising the gene set according to any of the preceding claims and possibly other means for real time PCR analysis.

5. A diagnostic kit or device comprising capture probes fixed upon a solid support and detecting specifically the expression of complementary target genes of the gene set according to any of the preceding claims and possibly others means for real time PCR.

6. The kit or device of claim 4 or 5 being a computerized system comprising
- a bio-assay signature configured for detecting a gene expression from a tumor sample based upon the gene set, kit or device according to any of the preceding claims, and
- a processor module configured to calculate expression of these genes and to generate a risk assessment for the tumor sample.

7. A method for a prognosis (prognostic) of cancer in mammal subject, preferably in a human patient, which comprises the step of measuring gene expression in a tumor sample obtained from a mammal subject, preferably a human patient, by putting into contact nucleotide sequences obtained from this tumor sample with the gene set, the kit or the device according to any of the preceding claims and possibly generating a prediction for worse prognosis and for benefit from targeted agents directed against the IGF-1 pathway the said tumor sample by designating the outcome of a IGF-1 specific regimen based on the IGF-1 (signature) gene set of claims 1 to 3.

8. The method of claim 7 wherein reduced expression of IGF-1 (signature) gene set predicts for a worse prognosis.

9. A targeted agent(s) directed against the IGF-1 pathway for use whether in a monotherapy or in combination with radiotherapy, chemotherapy, hormonotherapy or other targeted agents in the treatment of cancer having an activated IGF-1 pathway as measured by the method of claim 7 or 8 and/or by the gene set, kit or device according to claims 1 to 6.
